# EUROPEAN PATENT SPECIFICATION

(11) **EP 2 346 489 B1**
(45) Date of publication and mention of the grant of the patent: **15.08.2018**
(21) Application number: 08878044.0
(22) Date of filing: 07.11.2008
(51) Int. Cl.: A61K 8/9789, A61K 8/92, A61Q 19/00, A61K 8/362, A61Q 19/10

(54) **CLEANSING COMPOSITIONS**
REINIGUNGSZUSAMMENSETZUNGEN
COMPOSITIONS DE NETTOYAGE

(43) Date of publication of application: 27.07.2011
(73) Proprietor: Colgate-Palmolive Company, New York, NY 10022 (US)
(72) Inventor: ALVARADO, Emma, Somerville NJ 08876 (US); HARMALKER, Subhash, Somerset NJ 08873 (US)
(74) Representative: Wichmann, Hendrik
(86) International application number: PCT/US2008/082793
(87) International publication number: WO 2010/053488

(56) References cited:
- WO-A1-2004/105715
- US-A- 3 005 840
- US-A- 5 723 110
- US-A1- 2001 014 316
- US-A1- 2007 048 235
- US-B2- 6 780 825
- BEAL R E ET AL: "SOYBEAN SOAPSTOCK UTILIZATION: FATTY ACID ADDUCTS WITH ETHYLENE AND 1-BUTENE", JOURNAL OF THE AMERICAN OIL CHEMISTS' SOCIETY. (JAOCS) ED.2, SPRINGER, DE, vol. 52, no. 10, 1 January 1975 (1975-01-01), pages 400-403, XP001109540, ISSN: 0003-021X, DOI: 10.1007/BF02545274
- "New ingredients for personal cleansers", HAPPI HOUSEHOLD AND PERSONAL PRODUCTS INDUSTRY, RODMAN PUBLISHING, RAMSEY, NJ, US, 1 November 2003 (2003-11-01), XP009181478, ISSN: 0090-8878
- "food & flavor Applications note Restek Corporation @BULLET Determination of Omega-3 (n-3) and Omega-6 (n-6) Fatty Acid Composition in Evening Primrose Oil, Flax Seed Oil, Black Currant Oil, and Borage Oil", , 1 January 2000 (2000-01-01), XP055155292, Retrieved from the Internet: URL:http://www.restek.com/pdfs/59128.pdf [retrieved on 2014-11-26]

## Description

### BACKGROUND OF THE INVENTION

Providing moisture to skin, hair, or nails has been the goal of many products to prevent dryness and/or promote moisturization. Some products have used materials as a barrier to prevent the moisture within the body from escaping. Other products use materials to attract moisture to the skin.

It would be desirable to provide a composition that could provide a desired level of moisture along with desired effects on skin, hair, and/or nails.

### BRIEF SUMMARY OF THE INVENTION

A composition as defined in claim 1 comprising an adduct of an oil and an addition material and a flaxseed extract.

### DETAILED DESCRIPTION OF THE INVENTION

As used throughout, ranges are used as a shorthand for describing each and every value that is within the range. Any value within the range can be selected as the terminus of the range.

Unless indicated otherwise, the percentages specified herein are weight percentages based the active weight of the material in the total weight of the composition, which totals 100%.

The composition includes flaxseed extract, which includes proteins, omega-3 fatty acids, and omega-6 fatty acids. The flaxseed extract is available from Natunola Health, Inc. as NATUNOLA™ Flax Protein. Generally, the extract contains about 57 weight % omega-3 fatty acid and about 16 weight % omega-6 fatty acid. The ratio of omega-3 to omega-6 is 3.5 to 3.6. The amount of protein is 0.3 to 1% by weight.

The amount of flaxseed extract in the composition is 0.5 to 15% by weight. In other embodiments, the amount is 1, 2, 3, 4, 5, or at least 0.5 weight %. In other embodiments, the amount is less that 10, less than 5, less than 3, or less than 2 weight %. The amount of extract is based on the as supplied amount.

The composition includes an oil adduct synthesized by an oil and an addition material. Examples of oil adducts are described in U.S. Application Publication No. 2007/0048235 and U.S. Serial Nos. 60/990,801 and 60/990,818, both filed on 28 November 2007.

U.S. 3,005,840A discloses a composition comprising an adduct of a vegetable oil and maleic anhydride; and a material chosen from hydrolysed keratin, hydroxyethyl urea, and/or a quaternised nitrogen moisturising agent. WO 2004/105715A1 describes a cosmetic comprised of a carrier-matrix comprised of a substantially uniformly viscous liquid extract of flax seed. 'New ingredients for personal cleaners' (Happi household and personal products industry, Rodman Publishing, NJ, US, 1 November 2003) discloses a list of personal cleanser ingredients introduced by suppliers over the preceding 12 months. U.S. 2007/014316A1 discloses an organic oilbased liquid cleansing composition for the topical transdermal delivery of skin additives which has as its constituents, safflower oil, flaxseed oil, and tincture of benzoin in a glycerin base.

The oil adduct can be present In the composition In any desired amount of 1 to 3% by weight of the composition to give a desired level of moisturization.

In one embodiment, the oil adduct is a vegetable oil maleate, which is an adduct of a vegetable oil with maleic anhydride, that is castor oil maleate, which is available from ISP Corp. as CERAPHYL™ RMT.

In some embodiments, the oil adduct is synthesized from an oil which is capable of undergoing an adduct reaction. In some embodiments, the oil contains a hydroxyl group. A range of vegetable oils may be utilized for preparing the adduct including, for example, glycerin, in which the constituent hydroxyl groups are esterified with an equal or nearly equal number of fatty acid molecules. Many vegetable oils are triglycerides, *i.e.,* those glycerides in which three fatty acid molecules are chemically bonded to the glycerin backbone. Vegetable oils suitable for use in the present invention include, but are not limited to, almond, arachis (peanut), avocado, castor, coconut, corn, cottonseed, crambie, jojoba, lesquerella (bladder-pod), linseed, olive, palm, rapeseed (canola), rice bran, persic (apricot kernal), safflower, sesame, soybean, sunflower and veronia.

Differences in the functional properties of vegetable oils are generally attributed to variations in their respective fatty acid constituents. Different fatty acids may be present in vegetable oils suitable for use in the invention including, for example, myristic, licanic, 12-hydroxyoleic, eleostearic, ricinoleic, palmitic, stearic, oleic, linoleic, linolenic, arachidic, eicosenoic, behenic, erucic, palmitiolic, docosadienoic, lignoseric, tetracossenoic, margaric, margaroleic, gadoleic, caprylic, capric, lauric, pentadecanoic, and heptadecanoic acids. These fatty acid molecules can also vary in their degree of unsaturation and their hydroxyl groups can react like typical secondary alcohols by being eliminated or esterified.

In some embodiments, the oil is castor oil. Castor oil is one of three triglycerides that contain principally one fatty acid; it is about 90% ricinoleic acid, or 12-hydroxyoleic acid. The other two are tung oil, which is about 80% eleostearic acid, and oiticica oil, which is about 80% licanic acid. The hydroxyl group of the ricinoleic acid reacts like a typical secondary alcohol, i.e. it can be eliminated or esterified.

The addition material includes at least one member chosen from carboxylic acid anhydrides, maleic anhydride, dicarboxylic acids, fumaric acid, maleic acid, succinic acid, alpha hydroxy acids, beta hydroxy acids, lactic acid, glycolic acid, lactobionic acid, carnitine, salicylic acid, and (meth)acrylic acid.

When an alpha hydroxy acid or a beta hydroxy acid is selected, the composition provides an additional benefit. When the composition contacts the skin, the alpha hydroxy acid or a beta hydroxy acid can be liberated from the molecule by skin enzymes, such as esterases. The free acid then can act as an exfoliating agent in combination with the moisturization effect.

One example of a vegetable oil maleate is castoryl maleate, which maybe prepared by reacting castor oil with maleic anhydride to form the adduct as described in U.S. Patent No. 6,225,485 . In one embodiment, the castoryl maleate does not contain free maleic anhydride. The castoryl maleate adduct may be manufactured by reacting castor oil and cyclic carboxylic acid anhydride at a temperature of 75-120°C for an initial period of, for example, 4 to 24 hours, and then continuing the reaction at room temperature for at least one week to ensure that substantially all of the maleic anhydride has been reacted.

In another embodiment, the oil includes at least one member chosen from an ethoxylated oil, a hydrogenated oil, and an ethoxylated and hydrogenated oil. The average degree of ethoxylation in the ethoxylated (optionally hydrogenated) oil is less than about 10 based on moles of ethylene oxide per mole of oil.

In some embodiments, the oil may be hydrogenated or partially hydrogenated. Non-hydrogenated castor oil has an iodine value of 83-88. In certain embodiments, the oil may be hydrogenated to iodine values less than about 75. In some embodiments, the oil has an iodine value of about 25 to about 75. Iodine value can be measured by ASTM D5554-95 (2006).

According to some embodiments, the oil is ethoxylated. The ethoxylated oil may be partially hydrogenated or hydrogenated. As used throughout, the reference to the degree of ethoxylation is to average degree of ethoxylation of molecules in the sample based on the number of moles of ethylene oxide per mole of oil. The degree of ethoxylation may be about 1 to about 10. In some embodiments, the degree of ethoxylation may be about 1 to about 7. In other embodiments, the degree of ethoxylation may be about 1 to about 5. In other embodiments, the degree of ethoxylation may be about 3 to about 5. In other embodiments, the degree of ethoxylation can be 2, 3, 4, 5, 6, 7, 8, 9, or 10.

The combination of the adduct and the flax seed extract provides a moisture barrier to skin to help retain skin moisture. The adduct complexes with fatty acids to form a barrier that protects against external pollutants and allergens while retaining natural oils and moisture. Also, skin feel is changed from a slippery smooth feel to a squeaky clean feel during use, and after use, skin feels softer and smoother after drying. Additionally, the lather generated during washing is creamy, which is similar to the lather from bar soap.

The composition may also include one or more anionic surfactants, amphoteric surfactants, nonionic surfactants, cationic surfactants, and combinations thereof to form a cleansing composition. Those of ordinary skill in the art will be aware of suitable surfactants and other additives readily identifiable from the International Cosmetic Ingredient Dictionary and Handbook, 10th ed., (2004). Surfactants can be included in any desired amount. In one embodiment, surfactants are present in the composition in an amount of 0 to about 40% by weight. In one embodiment, the surfactants are present in an amount of about 1 to about 40% by weight. In one embodiment, surfactants are present in the composition in an amount of about 5 to about 40% by weight. In one embodiment, the surfactants are present in an amount of about 1 to about 10% by weight.

A variety of anionic surfactants can be utilized in the moisturizing body wash composition including, for example, long chain alkyl (C₆-C₂₂) materials such as long chain alkyl sulfates, long chain alkyl sulfonates, long chain alkyl phosphates, long chain alkyl ether sulfates, long chain alkyl alpha olefin sulfonates, long chain alkyl taurates, long chain alkyl isethionates (SCI), long chain alkyl glyceryl ether sulfonates (AGES), sulfosuccinates and the like. These anionic surfactants can be alkoxylated, for example, ethoxylated, although alkoxylation is not required. These surfactants are typically highly water soluble as their sodium, potassium, alkyl and ammonium or alkanol ammonium containing salt form and can provide high foaming cleansing power. Other equivalent anionic surfactants may be used. In one embodiment, the anionic surfactant comprises sodium laureth sulfate, sodium pareth sulfate, and combinations thereof. Anionic surfactants can be included in any desired amount. In one embodiment, anionic surfactants are present in the composition in an amount of 0 to about 15% by weight. In one embodiment, anionic surfactants are present in an amount of about 6 to about 8% by weight.

Amphoteric surfactants may also be included in the composition. These surfactants are typically characterized by a combination of high surfactant activity, lather forming and mildness. Amphoteric surfactants include, but are not limited to, derivatives of aliphatic secondary and tertiary amines in which the aliphatic radical can be straight chain or branched and wherein one of the aliphatic substituents contains about 8 to about 18 carbon atoms and one contains an anionic water solubilizing group, e.g., carboxy, sulfonate, sulfate, phosphate, or phosphonate. Examples of such compounds include sodium 3-dodecyaminopropionate, sodium 3-dodecylaminopropane sulfonate, N-alkyl taurines and N-higher alkyl aspartic acids. Other equivalent amphoteric surfactants may be used. Examples of amphoteric surfactants include, but are not limited to, a range of betaines including, for example, high alkyl betaines, such as coco dimethyl carboxymethyl betaine, lauryl dimethyl carboxy-methyl betaine, lauryl dimethyl alpha-carboxyethyl betaine, cetyl dimethyl carboxymethyl betaine, lauryl bis-(2-hydroxyethyl)carboxy methyl betaine, stearyl bis-(2-hydroxypropyl)carboxymethyl betaine, oleyl dimethyl gamma-carboxypropyl betaine, and lauryl bis-(2-hydroxypropyl)alpha-carboxyethyl betaine, sulfobetaines such as coco dimethyl sulfopropyl betaine, stearyl dimethyl sulfopropyl betaine, amido betaines, amidosulfobetaines and the like. Betaines having a long chain alkyl group, particularly coco, may be particularly useful as are those that include an amido groups such as the cocamidopropyl and cocoamidoethyl betaines. Amphoteric surfactants can be included in any desired amount. In one embodiment, amphoteric surfactants are present in the composition in an amount of 0 to about 15% by weight. In one embodiment, the amphoteric surfactants are present in the composition in an amount of about 4 to about 6% by weight.

Examples of nonionic surfactants include, but are not limited to, polysorbate 20, long chain alkyl glucosides having C₈-C₂₂ alkyl groups; coconut fatty acid monoethanolamides such as cocamide MEA; coconut fatty acid diethanolamides, fatty alcohol ethoxylates (alkylpolyethylene glycols); alkylphenol polyethylene glycols; alkyl mercaptan polyethylene glycols; fatty amine ethoxylates (alkylaminopolyethylene glycols); fatty acid ethoxylates (acylpolyethylene glycols); polypropylene glycol ethoxylates (for example the PLURONIC™ block copolymers commercially available from BASF); fatty acid alkylolamides, (fatty acid amide polyethylene glycols); N-alkyl-, N-alkoxypolyhydroxy fatty acid amides; sucrose esters; sorbitol esters; polyglycol ethers; and combinations thereof. Nonionic surfactants can be included in any desired amount. In one embodiment, nonionic surfactants are present in the composition in an amount of 0 to about 3% by weight. In one embodiment, nonionic surfactants are present in the composition in an amount of about 0.5 to about 1.5% by weight.

Cationic surfactants can also be included in the composition. Examples of cationic surfactants include, but are not limited to any quaternium or polyquaternium compound. Cationic surfactants can be included at any desired level. In one embodiment, cationic surfactants are present in the composition in an amount of 0 to about 2 % by weight. In one embodiment, cationic surfactants are present in the composition in an amount of about 0.1 to about 0.3 % by weight.

Many additional surfactants are described in McCUTCHEON'S DETERGENTS AND EMULSIFIERS (1989) and other reference materials that are well known to those of ordinary skill in the art.

Additionally, a suspending agent can be included to structure the surfactant to aid in suspending particles. Suspending agents are any material that increases the ability of the composition to suspend material. Examples of suspending agents include, but are not limited to, synthetic structuring agents, polymeric gums, polysaccharides, pectin, alginate, arabinogalactan, carrageen, gellan gum, xanthum gum, guar gum, rhamsan gum, furcellaran gum, and other natural gum. A synthetic structuring agent in one embodiment is a polyacrylate. One acrylate aqueous solution used to form a stable suspension of the solid particles is manufactured by Lubrizol as CARBOPOL™ resins, also known as CARBOMER™, which are hydrophilic high molecular weight, crosslinked acrylic acid polymers. In one embodiment, the polymer is CARBOPOL™ Aqua SF-1. Other polymers that can be used include, but are not limited to, CARBOPOL™ Aqua 30, CARBOPOL™ 940 with a molecular weight of approximately 4,000,000, and CARBOPOL™ 934 with a molecular weight of approximately 3,000,000.

The suspending agents can be used alone or in combination. The amount of suspending agent can be any amount that provides for a desired level of suspending ability. In one embodiment, the suspending agent is present in an amount of about 0.01 to about 15% by weight of the composition. In other embodiments, the amount of suspending agent is about 1% to about 10%.

In another embodiment, glycerin may be included in the composition. The glycerin can be included in any desired amount to provide a desired level of moisturization. In one embodiment, the glycerin is present in an amount of 0 to about 8% by weight. In other embodiments, the glycerin can be present at about 1, about 1.5, about 2, about 2.5, about 3, about 3.5, about 4, about 4.5, about 5, about 5.5, about 6, about 6.5, about 7, or about 7.5% by weight.

In another embodiment, the composition can contain vitamin E and/or vitamin E acetate (a vitamin E precursor). In one embodiment, the amount of vitamin E and/or vitamin E acetate in the composition is about 0.005 weight % to about 0.5 weight %. In other embodiments, the amount is at least 0.01, at least 0.05, or at least 0.1 weight %. In other embodiments, the amount is less than 0.5 weight %. For more information about vitamin E and/or vitamin E acetate, see United States Patent No. 6,730,310 to Nabi et al.

In another embodiment, the composition may also contain creatine. Creatine can be used to support the energy cycle in skin cells. Creatine can be included at any desired amount to achieve any desired level of energy support in cells. In one embodiment, the creatine is present in the composition in an amount of 0 to about 2% by weight.

In another embodiment, the composition can further include a moisturizing agent chosen from a hydrolyzed keratin, hydroxyethyl urea, and/or a quaternized nitrogen moisturizing agent. Any one of these can be used alone, or any combination of these materials can be used.

In another embodiment, a hydrolyzed keratin can be present in the composition. Any hydrolyzed keratin can be included in the composition. In one embodiment, the hydrolyzed keratin comprises an extract of goat hair. In one embodiment, the goat hair is cashmere. The hydrolyzed keratin can be present in the composition in any desired amount to give a desired level of moisturization. In one embodiment, the hydrolyzed keratin is present in an amount of 0 to about 0.005% by weight. In another embodiment, the hydrolyzed keratin is present in an amount of about 0.0005 to about 0.005% by weight. In another embodiment, the hydrolyzed keratin is present at about 0.0015% by weight.

In another embodiment, hydroxyethyl urea can be present in the composition. The hydroxyethyl urea can be present in the composition in any desired amount to give a desired level of moisturization. In one embodiment, the hydroxyethyl urea is present in an amount of 0 to about 13% by weight, In one embodiment, the hydroxyethyl urea is present at about 6% by weight.

In another embodiment, a quaternary nitrogen moisturizing agent can be present in the composition. The quaternary nitrogen moisturizing agent is a moisturizing agent that contains a quaternary nitrogen in its structure. Examples of quaternary nitrogen moisturizing agents include, but are not limited to, hydroxypropyl bis-hydroxyethyldimonium chloride (available as COLA™Moist 200 from Colonial Chemicals, Inc.) having the structure 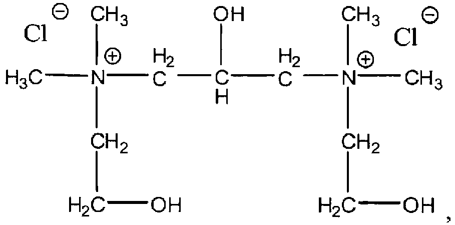 a material having the structure 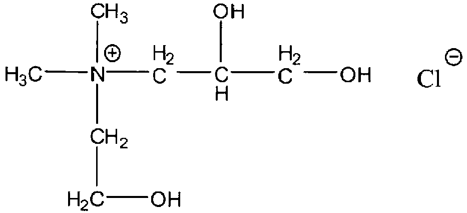 (which is described in United States Patent No. 6,869,977, a choline salt (which is described in United States Patent Nos. 6,475,965 and 6,265,364) carnitine, and combinations thereof. Naturally occurring carnitine is L-carnitine. The quaternary nitrogen moisturizing agent can be present in the composition in any desired amount to give a desired level of moisturization. In one embodiment, the quaternary nitrogen moisturizing agent is present in an amount of 0 to about 5. In another embodiment, the quaternary nitrogen moisturizing agent is present in an amount of about 0.1 to about 1% by weight. In another embodiment, the quaternary nitrogen moisturizing agent is present at about 1% by weight.
In another embodiment, skin compatible oils can be included in the composition. Skin compatible oils include a range of liquid hydrocarbons, for example, linear and branched oils such as liquid paraffin, squalene, squalane, mineral oil, low viscosity synthetic hydrocarbons such as polyalphaolefins, commercially available from ExxonMobil under the trade name PURESYN PAO and polybutene under the trade name PANALANE™ or INDOPOL™. Light (low viscosity) highly branched hydrocarbon oils may also be suitable in some instances. Other useful skin compatible oils may be silicone based, for example, linear and cyclic polydimethyl siloxane, organo functional silicones (alkyl and alkyl aryl), and amino silicones.
In one embodiment, a moisturizing body wash composition also utilizes, as a thickening agent, a blend of PEG-150 distearate and PPG-2 hydroxyethyl cocamide for countering a decrease in viscosity associated with the concentrations of moisturizing agents utilized in some embodiments of the moisturizing body wash composition. This blended thickening agent allows the composition to achieve viscosities beyond those that could be achieved with conventional thickening agents, for example sodium chloride alone, and is able to achieve suitable viscosities at relatively low concentrations, The relatively low concentrations used to achieve the desired viscosities are also advantageous with respect to manufacturing processes that may be employed to manufacture the moisturizing body wash composition, thereby reducing the need for larger equipment or modifications and the capital expenditure associated with manufacturing the moisturizing body wash composition if other thickening agents were used. The PEG-150 distearate and the PPG-2 hydroxyethyl cocamide can be present in any amount to achieve a desired viscosity. In one embodiment, the amount of PEG-150 distearate in the composition is 0 to about 2% by weight. In one embodiment, the amount of PPG-2 hydroxyethyl cocamide in the composition is 0 to about 2% by weight. In one embodiment, the weight ratio of the PEG-150 distearate to the PPG-2 hydroxyethyl cocamide can be about 3:1 to about 1:3. In one embodiment, the PEG-150 distearate and the PPG-2 hydroxyethyl cocamide are each present at 0.0225% by weight. The PEG-150 distearate and the PPG-2 hydroxyethyl cocamide are available as a mixture from Uniqema under the trade name PROMIDIUM™ LTS.

In another embodiment, the composition can contain suspended material. The suspended material can be beads. In one embodiment, the beads are available from ISP Corp. as CAPTIVATES™ HC4567, HC4451, HC4590, HC, 4635, HC4637, or HC4638, .

In other embodiments, the composition may include any of following materials in any desired amount to achieve a desired effect in the composition (amounts that can be used in some embodiments are provided): one or more alkaline salts, for example, sodium chloride, sodium sulfate, sodium carbonate, sodium bicarbonate and/or their equivalents (0 to 5% by weight); foaming agents, for example decyl glucoside, and/or their equivalents (0 to 3% by weight); glyceryl esters and derivatives, for example glycol distearate, and/or their equivalents(0 to 3%; by weight); sequestrants, for example, tetrasodium EDTA, and/or their equivalents (0 to 2% by weight); biocides, for example, Triclosan (2,4,4'-trichloro-2'-hydroxydiphenyl ether), DMDM hydantoin, formaldehyde and/or imidazolidinyl urea, and/or their equivalents (0 to 2% by weight); organic acids, for example, citric acid and/or formic acid and/or their equivalents (0 to 2% by weight); viscosity modifiers (0 to 2% by weight); fragrances and/or perfumes (0 to 5% by weight); preservatives, for example, phenoxyethanol, formaldehyde solution, parabens, pentanediol or sorbic acid (0 to 2% by weight); pearlizing agents, for example, glycol distearic esters, such as ethylene glycol distearate, but also fatty acid monoglycol esters (0 to 3% by weight); stabilizers, for example, metal salts of fatty acids, such as e.g., magnesium stearate, aluminum stearate and/or zinc stearate (0 to 2% by weight); and dyes and pigments that are approved and suitable for cosmetic purposes.

Water may be included in the composition. Water can be included in an amount of 0 to about 95% by weight. In one embodiment, water is present at about 50% to about 90% by weight.

The composition can be used to moisturize skin, hair, and/or nails. The composition is applied to skin, hair, and/or nails. If the composition is a rinse off composition, the composition is rinsed off. The composition can be left on for any desired amount of time. The composition can be included in any product that contacts skin, including hair, and/or nails. The composition can be in the form of a body wash, a shower gel, a hand wash, a soap bar, a shampoo, a conditioner, a dishwashing liquid, a skin lotion, a sunscreen, a bubble bath, an oral care product, a dentifrice, a toothpaste, a mouthwash, an antiperspirant, a deodorant, or a foot soak.

The composition can also be used to apply a substance to a substrate. The substance is included in the composition, and the composition is applied to a substrate. The substrate can be any desired substrate. In one embodiment, the substrate can be skin, hair, and/or nails. The substance can be any substance that is attracted to the composition. In one embodiment, the substance is chosen from fragrances, sunscreen, pigments, insect repellents, and/or hydrophobic materials.

The following examples are merely illustrative, are not in accordance with the invention and do not in any way limit the scope of the invention as described and claimed. Unless otherwise listed, the amount are % by weight of the composition based on the active weight of the material.

### Example 1

The following composition was prepared by mixing of the ingredients. The amounts are based on the as supplied amounts.

| Material | Weight % |
|---|---|
| Surfactant Base | 82 |
| 71.35 % water | |
| 12.4% alkyl ether sulfate surfactant (Steol™ OS-270) | |
| 10.6% Cocamidopropyl betaine | |
| 2.6% Polyquaternium-7 | |
| 2.4% Decyl glucoside | |
| 0.4% DMDM Hydantoin | |
| 0.2% Tetrasodium EDTA (39% solution) | |
| 0.05% Formalin | |
| Cocamidopropyl betaine | 8 |
| Castor Oil Maleate (Ceraphyl™ RMT) | 0.55 |
| Glycerin | 6 |
| Flaxseed extract (Natunola™ Flax Protein) | 1 |
| DMDM Hydantoin | 0.1 |
| DI Water | 0.9 |
| Shea butter/almond oil blend | 0.05 |
| Promidium LTS PEG-150 distearate and the PPG-2 hydroxyethyl cocamide | 0.25 |
| Fragrance | 1 |
| Vitamin E acetate | 0.1 |
| Citric acid solution (50% solution) | 0.1 |

Formulas A to E are for body wash compositions.

| Material (weight %) | A | B | C | D | E |
|---|---|---|---|---|---|
| Water and minors to make 100% (target water) | Q.S. (77.7) | Q.S. (77.9) | Q.S. (81.5) | Q.S. (80.1) | Q.S. (80.1) |
| C10-16 alcohol ethoxylate, sodium sulfate | 7 | 7 | 8.8 | 8.3 | 8.3 |
| glycerin | 6 | 6 | 3 | 3.3 | 3.3 |
| Cocamidopropyl betaine | 5 | 5 | 1.6 | 2.8 | 2.8 |
| Sodium Chloride | 1.1 | 1.1 | 0.5 | 0.8 | 0.8 |
| Fragrance | 0.8 | 0.8 | 0.8 | 1 | 1 |
| Alkyl polyglucoside | 0.7 | 0.7 | 0 | 0 | 0 |
| Castor oil maleate (CERAPHYL™ RMT) | 0.55 | 0.55 | 0.55 | 0.3 | 0.3 |
| DMDM Hydantoin | 0.3 | 0.3 | 0.3 | 0.3 | 0.3 |
| Lauryl polyglucose | 0.3 | 0.3 | 0 | 0 | 0 |
| Polyquaternium-7 | 0.2 | 0.2 | 0 | 0 | 0 |
| Vitamin E acetate | 0.1 | 0 | 0 | 0.1 | 0.1 |
| Citric Acid | 0.1 | 0 | 0 | 0 | 0 |
| Tetrasodium EDTA | 0.1 | 0.1 | 0.1 | 0.1 | 0.1 |
| Flaxseed extract (Natunola™ Flax protein) | 1 | 1 | 1 | 1 | 1 |
| Carbopol™ Aqua SF-1 acrylates polymer | 0 | 0 | 2.5 | 2.4 | 2.4 |
| Mineral Oil | 0 | 0 | 0 | 0.3 | 0 |
| ISP Captivates™ HC4567 beads | 0 | 0 | 0 | 0.3 | 0 |
| ISP Captivates™ HC4451 beads | 0 | 0 | 0 | 0 | 0.3 |

Formulas F-J are additional body wash compositions

| Material (weight %) | F | G | H | I | J |
|---|---|---|---|---|---|
| Water and minors to make 100% (target water) | Q.S. (78.8) | Q.S. (78.7) | Q.S. (78.7) | Q.S. (78.7) | Q.S. (78.7) |
| C10-16 alcohol ethoxylate, sodium sulfate | 8.3 | 8.3 | 8.3 | 8.3 | 8.3 |
| glycerin | 3.3 | 3.2 | 3.2 | 3.2 | 3.2 |
| Cocamidopropyl betaine | 2.8 | 2.8 | 2.8 | 2.8 | 2.8 |
| Sodium Chloride | 0.8 | 0.8 | 0.8 | 0.8 | 0.8 |
| Fragrance | 0.9 | 1 | 1 | 1 | 1 |
| Castor oil maleate (CERAPHYL™ RMT) | 0.3 | 0.3 | 0.3 | 0.3 | 0.3 |
| DMDM Hydantoin | 0.3 | 0.3 | 0.3 | 0.3 | 0.3 |
| Vitamin E acetate | 0.1 | 0.1 | 0.1 | 0.1 | 0.1 |
| Tetrasodium EDTA | 0.1 | 0.1 | 0.1 | 0.1 | 0.1 |
| Flaxseed extract (Natunola™ Flax Extract 130) | 1 | 1 | 1 | 1 | 1 |
| Carbopol™ Aqua SF-1 acrylates polymer | 2.4 | 2.4 | 2.4 | 2.4 | 2.4 |
| Arlasilk™ phospolipid efa (linoleamidopropyl PG-dimonium chloride phosphate) | 0.1 | 0.1 | 0.1 | 0.1 | 0.1 |
| ISP Captivates™ HC4567 beads | 0.25 | | 0.25 | | |
| ISP Captivates™ HC4590 beads | 0.25 | | 0.25 | 0.25 | |
| ISP Captivates™ HC4637 beads | | 0.25 | | 0.25 | 0.25 |
| ISP Captivates™ HC4638 beads | | 0.25 | | | |
| ISP Captivates™ HC4635 beads | | | | | 0.25 |

Formula K is for a foaming liquid hand soap composition.

| Material (weight %) | K |
|---|---|
| Water and minors to make 100% (target water) | Q.S. (88.4) |
| sodium laureth sulfate (2EO) | 7.6 |
| Cocamidopropyl betaine | 1.8 |
| Fragrance | 0.4 |
| Castor oil maleate (CERAPHYL™ RMT) | 0.1 |
| DMDM Hydantoin | 0.3 |
| PEG-120 Methyl glucose dioleate | 0.15 |
| Tetrasodium EDTA | 0.1 |
| Flaxseed extract (Natunola™ Flax Extract 130) | 1 |

The compositions can be made by mixing of the ingredients. For ease of manufacture, the surfactants, acrylates polymers, and minors can be prepared as a base material that can be formulated to specific compositions by adding additional materials (such as fragrance, castor oil maleate, flaxseed extract, cocamidopropyl betaine, glycerin, DMDM hydantoin, salt, and beads) and mixing.

## Claims

1. A composition comprising:
an adduct of an oil comprising castor oil maleate in an amount of 1 to 3% by weight of the composition ;
a flaxseed extract in an amount of 0.5 to 15% by weight of the composition,
wherein the flaxseed extract comprises 0.3 to 1% by weight of proteins, omega-3 fatty acids and omega-6 fatty acids, wherein a weight ratio of the omega-3 fatty acids to the omega-6 fatty acids is 3.5:1 to 3.6:1.

2. The composition of claim 1 further comprising a surfactant.

3. The composition of claim 2, wherein the surfactant is present in an amount of 1 to 40 weight % percent based on a total weight of the composition.

4. The composition of claim 2, wherein the surfactant comprises at least one surfactant chosen from anionic surfactants, amphoteric surfactants, and non-ionic surfactants.

5. The composition of claim 1 further comprising glycerin.

6. The composition of claim 1 further comprising creatine.

7. The composition of claim 1 further comprising PEG-150 distearate and PPG-2 hydroxyethyl cocamide.

8. The composition of claim 1, further comprising about 1 to about 7 to about 15 weight % surfactant.

9. A method comprising applying the composition of claim 1 to skin, hair, and/or nails.

## Patentansprüche

1. Zusammensetzung umfassend:
ein Addukt eines Öls, welches Kastorölmaleat umfasst, in einer Menge von 1 bis 3% bezogen auf das Gewicht der Zusammensetzung;
ein Leinsamenextrakt in einer Menge von 0,5 bis 15%, bezogen auf das Gewicht der Zusammensetzung,
wobei das Leinsamenextrakt 0,3 bis 1%, bezogen auf das Gewicht Proteine, Omega-3 Fettsäuren und Omega-6 Fettsäuren umfasst,
wobei ein Gewichtsverhältnis von Omega-3 Fettsäuren zu Omega-6 Fettsäuren 3,5:1 bis 3,6:1 beträgt.

2. Zusammensetzung nach Anspruch 1, des Weiteren umfassend ein oberflächenaktives Mittel.

3. Zusammensetzung nach Anspruch 2, wobei das oberflächenaktive Mittel in einer Menge von 1 bis 40 Gew.-%, basierend auf dem Gesamtgewicht der Zusammensetzung vorliegt.

4. Zusammensetzung nach Anspruch 2, wobei das oberflächenaktive Mittel mindestens ein oberflächenaktives Mittel, ausgewählt aus anionischen oberflächenaktiven Mitteln, amphoterischen oberflächenaktiven Mitteln, und nicht-ionischen oberflächenaktiven Mitteln umfasst.

5. Zusammensetzung nach Anspruch 1, des Weiteren umfassend Glycerol.

6. Zusammensetzung nach Anspruch 1, des Weiteren umfassend Kreatin.

7. Zusammensetzung nach Anspruch 1, des Weiteren umfassend PEG-150 Distearat und PPG-2 Hydroxyethylcocamid.

8. Zusammensetzung nach Anspruch 1, des Weiteren umfassend etwa 1 bis etwa 7 bis etwa 15 Gew.% oberflächenaktives Mittel.

9. Verfahren umfassend das Anwenden der Zusammensetzung gemäß Anspruch 1 an Haut, Haar, und/oder Nägeln.

## Revendications

1. Composition comprenant :
un adduit d'une huile comprenant du maléate d'huile de ricin en une quantité de 1 à 3 % en poids de la composition ;
un extrait de graines de lin en une quantité de 0,5 à 15 % en poids de la composition,
dans lequel l'extrait de graines de lin comprend 0,3 à 1 % en poids de protéines, d'acides gras oméga-3 et d'acides gras oméga-6, dans lequel un rapport pondéral des acides gras oméga-3 aux acides gras oméga-6 est de 3,5:1 à 3,6:1.

2. Composition selon la revendication 1, comprenant en outre un tensioactif.

3. Composition selon la revendication 2, dans laquelle le tensioactif est présent en une quantité de 1 à 40 % en poids par rapport au poids total de la composition.

4. Composition selon la revendication 2, dans laquelle le tensioactif comprend au moins un tensioactif choisi parmi les tensioactifs anioniques, les tensioactifs amphotères et les tensioactifs non ioniques.

5. Composition selon la revendication 1, comprenant en outre de la glycérine.

6. Composition selon la revendication 1, comprenant en outre de la créatine.

7. Composition selon la revendication 1, comprenant en outre du distéarate de PEG-150 et du PPG-2 hydroxy-éthyl cocamide.

8. Composition selon la revendication 1, comprenant en outre environ 1 à environ 7 à environ 15 % en poids de tensioactif.

9. Procédé comprenant l'application de la composition selon la revendication 1 sur la peau, les cheveux et/ou les ongles.
